# EUROPEAN PATENT SPECIFICATION

(11) **EP 1 779 772 B1**
(45) Date of publication and mention of the grant of the patent: **03.06.2009**
(21) Application number: 05762308.4
(22) Date of filing: 21.06.2005
(51) Int. Cl.: A61B 5/00, G08B 21/00, G08B 23/00, G08B 25/10, G01P 15/00

(54) **SYSTEM FOR ANALYSING A PERSON'S ACTIVITY AND FOR AUTOMATIC FALL DETECTION**
SYSTEM ZUR AUSWERTUNG DER AKTIVITÄT EINER PERSON UND ZUM AUTOMATISCHEN NACHWEIS VON STÜRZEN
SYSTEME D'ANALYSE DE L'ACTIVITE D'UNE PERSONNE ET DETECTION AUTOMATIQUE DE CHUTES

(30) Priority: 21.06.2004 ES 200401513 P
(43) Date of publication of application: 02.05.2007
(73) Proprietor: FUNDACION FATRONIK, 20009 San Sebastian, Guipuzcoa (ES); Instituto Gerontologico Matia, S.L.U. (Ingema), 20002 San Sebastian (ES)
(72) Inventor: AZCOITIA ARRECHE, Jose, Miguel, E-20870 Elgoibar (Guipuzcoa) (ES); EIZMENDI LORIZ, Gorka, E-20870 Elgoibar (Guipuzcoa) (ES); PEROLLE, Guillaume, E-20870 Elgoibar (Guipuzcoa) (ES); SANCHEZ FUENTES, David, E-20870 Elgoibar (Guipuzcoa) (ES); YANGUAS LEZAUN, Jose, Javier, E-20002 San Sebastian (ES)
(74) Representative: Carpintero Lopez, Francisco
(86) International application number: PCT/ES2005/000349
(87) International publication number: WO 2006/000605

(56) References cited:
- FR-A- 2 760 116
- GB-A- 2 323 196
- US-A1- 2001 048 368
- US-B1- 6 208 251

## Description

### OBJECT OF THE INVENTION

The object of the invention is a portable and wireless system for detecting falls, with a high reliability as it allows detecting only real falls, discarding false alarms due to strange or unexpected movements of the user but which do not entail any risk.

The object of the invention is the methodology used to detect and discriminate falls, as well as the algorithms implemented for executing it.

Also object of the invention are the units that allow detecting falls, generating alarms and transmitting the corresponding messages to the remote control centre, as well as detecting the user's position in both closed premises and open spaces.

The system of the invention also allows analysing a person's activity and a remote monitoring, determining the type or level of activity developed.

### BACKGROUND OF THE INVENTION

Currently many systems are appearing to allow a remote control of persons, with a particular emphasis on detecting risk situations in older people or those who live alone, for example detecting possible falls or situations of risk to the person.

Remote communication devices have been used for some time comprising an emitter placed in the user's home or which can be carried by the person, provided with a button that is actuated by the user to send an alarm signal to a rescue station in case of emergency. These systems have the great disadvantage that they require the user's actuation, so that they are not effective if the user cannot activate the alarm signal because he/she is unconscious, is not near the emitter or for any other reason.

For this reason, new devices are being developed that are able to detect automatically a risk situation, and specifically falls. These systems generally incorporate an accelerometer that can detect the user's accelerations and automatically emit alarm signals to the control centres.

Communication between the emitter and the receiver placed in the remote control centre can be by radio, via the land telephone line, wireless telephone, satellite or Internet.

In addition, some systems incorporate in the emitter a global positioning system (GPS) that allow knowing the exact position of the user.

The following patents can be cited by way of example of this type of system:

### US Patent 3,163,856 in the name of Frederick G. Kirby

This patent describes an alarm device that is activated when a person or object stops moving for a certain time. The device emits a radiofrequency signal each time the user's wrist moves, generating an alarm when the user's wrist stops moving.

### US Patent 4,110,741 in the name of Societe Chimique des Charbonnages

This patent describes a device able to monitor the physical activity of persons and to generate an alarm when the person remains static due to a fall or the like. The device comprises a motion detector based on an accelerometer that constantly monitors the normal movements of the person, transmitting a signal to a receiver.

If the person stops moving for some time, the device emits an audible alarm and after a time stops transmitting to the receiver, which activates an alarm.

### European Patent 0849 715 in the name of GGT Gesellschaft Fur Gerontotechnik, mbH

This patent claims a fall detection method incorporating monitoring means placed on the upper part of the user's body which determine, in addition to position and movement values corresponding to changes in the position and inclination of the person, values of speed, acceleration and impact with an inclination and movement sensor.

### European Patent 0877 346 in the name of CSEM

This patent corresponds to a device that monitors the activity and detects falls of a person, comprising means for establishing a probability factor that represents the likelihood of a risk situation based on the variation of the position, acceleration and velocity signals.

### US Patent 6,160,478 in the name of Sarcos LC

This patent relates to a system for monitoring the physical activity of a person comprising a multidimensional accelerometer that generates a signal corresponding to the acceleration and processing means to convert the accelerometer signal into data that are analysed until a pulse is received indicating a possible fall, after which the importance of the fall is determined by the magnitude of the pulse.

The system also incorporates communication means for sending the data to a remote position and communication means in the remote position for receiving the transmitted data.

### US Patent 6,433,690 in the name of Sarcos LC

This patent is a continuation of US 6,160,478 and relates to a method for monitoring a person's fall based on sampling the output signal of an accelerometer that indicates the acceleration and angle of the body, determining a possible fall based on the body angle and the duration of the fall, determining that an uncontrolled fall has occurred when the duration measured is less than a predefined time and determining that it is an important fall when the change of body angle and the magnitude of the acceleration exceed a certain threshold.

### US Patent 6,307,481 in the name of Ilife Systems, Inc.

This patent claims a system that determines the movement of a body in relation to its surroundings and comprises a sensor that detects the static and dynamic acceleration associated to a body, and a processor associated to the sensor that processes static and dynamic acceleration to determine whether the body motion is within predefined margins.

### US Patent 6,703,939 in the name of Ilife Solution

This patent is a continuation of US Patent 6,307,481; it claims a system for determining the motion of a body with similar characteristics to the main patent, but in which the sensor measures the acceleration along three axis of reference, comprising a plurality of devices for measuring acceleration.

### US Patent 6,501,380 in the name of Ilife Solution

This patent is also a continuation of US Patent 6,307,481. In this case it protects a system as that of the initial Patent, incorporated in a communications system. This is, it is a system incorporating a sensor to measure the static and dynamic acceleration, a processor that determines whether the movement is within the predetermined range and a communication device which, according to the text of the Patent, can be among others a cellular telephone.

### US Patent 6,661,347 in the name of Ilife Solution

This patent is also a continuation of US Patent 6,307,481. In this case it protects a system as that of the initial Patent, incorporated in position location system. This is, it is a system incorporating a sensor to measure the static and dynamic acceleration, a processor that determines whether the movement is within the predetermined range and a positioning device which, according to the text of the Patent, can be a global positioning system (GPS) device. This positioning device can communicate via a wireless network, a cable network or via the Internet.

### US Patent Application 10/331,958 (Pub. No. 2003/0146844) in the name of Ilife Solution

This patent is a continuation of US Patent 6,501,386, which in turn is a continuation of US Patent 6,307,481. It relates to a system incorporated in communications devices that is able to determine the movements of a body comprising a sensor associated to a body that measures its acceleration, and a processor associated to the sensor that analyses the acceleration detected and determines whether the motion of the body is within acceptable ranges.

Document GB-A-2 323 196 discloses a device comprising an accelerometer detecting acceleration values along a vertical and a horizontal axis, and a processor which determines falls under two different conditions, one where the acceleration exceeds a maximum limit, and another one where the acceleration exceeds another lower limit, wherein these limits are set to be customised to the user.

However, despite the numerous efforts made in the field, there are currently very few devices available in the market, and those that are available have great reliability problems, mainly for detecting falls as most generate many false alarms, so that it can be said that the definitive fall detector is yet to be developed.

### DESCRIPTION OF THE INVENTION

The invention is defined in claims 1 and 6.

The system of the invention allows solving the problems described above providing a portable and wireless system for detecting falls with a great reliability, as it can discard efficiently false alarms due to strange or unexpected movements of the user which do not entail any risk.

The portable device allows detecting falls by the use of an accelerometer that can detect the movements of the user along the vertical axis X and the horizontal axis Y, the system being completed by a microprocessor that includes activity level and fall detection algorithms, as well as a memory that allows storing predetermined values used as limits for detecting falls. The vertical axis X is understood as a vertical axis when the user is standing, and the horizontal axis Y is a horizontal axis when the user is standing.

The system also incorporates a wireless communications module and a global positioning and location device, such as GPS, for determining the position of the person.

The system of the invention is based on a direct detection of when the instantaneous acceleration along the Y axis exceeds a maximum limit value, determining the unambiguous existence of a fall and simultaneously a differential detection when the instantaneous acceleration along the X and Y axis exceeds differential values without reaching the maximum limit values, determining in this case a possible fall.

The direct detection considers the acceleration generated by the impact of the person against the ground, as in the moment of impact the acceleration generated is so high that it exceeds the maximum limits and it is considered that a fall has occurred with certainty.

Differential detection allows detecting gentler falls or falls in two stages. In this case, a possible fall is detected by the value of the acceleration generated by the impact of the body against the ground which, although it may not exceed the maximum values, is high enough to allow considering that an important event has occurred that may be a fall, as it exceeds the differential values.

In this case, a more complex detection method is used as it is not possible beforehand to ensure that a fall has occurred nor to generate false alarms continuously. For this reason, when these acceleration variations are detected the state after the variation is analysed in detail in order to verify that the fall has occurred before generating the alarm signal.

To do so, it is checked that the activity of the person after the variation is very limited, as is typically the case after an important fall, in this case checking that the instantaneous acceleration values are within predetermined resting limits for both the X and Y axis.

Another critical factor to analyse in this case is the position of the person after the variation in the acceleration detected. To do so, the method of the invention analyses the orientation of the person with respect to the gravitational force.

To establish the maximum and differential limits, the detection method of the invention is based on a continuous measurement of the acceleration along the X and Y axis in the two seconds prior to the possible fall, these values being used to calculate an average value of the acceleration that are used to determine the maximum, differential and resting limit values that are stored in the memory of the device.

After detecting a fall, whether directly or differentially, an alarm signal is triggered that can be cancelled by the user during a certain time, after which if it is not cancelled an alarm message will be sent to the remote control station alerting of the fall and indicating the place in which it has occurred.

The alarm signal is sent by wireless GSM, GPRS or UMTS type telephony to a remote receiver station, so that the device can even be used in open spaces as no fixed receivers are needed to transmit the signal. In addition to the alarm, the microprocessor also sends the position data obtained from the global positioning locator, allowing to locate the place of the fall with great accuracy even if users are far from their usual locations.

The system also allows a continuous and remote monitoring of the physical activity and/or mobility of the persons for subsequent evaluations of wellness and quality of life.

To do so, the acceleration data recorded continuously by the accelerometer are processed by the microprocessor using an algorithm that allows identifying the type of activity being performed by the person among a number of predetermined activities stored in the system memory.

The information obtained is stored in the memory of the device and is periodically sent in a summarised form to the control centre via the GSM/GPRS/UMTS module.

In this way, the control centre uses the information received to evaluate the mobility of the person and thereby can periodically monitor the physical progress.

### DESCRIPTION OF THE DRAWINGS

To complete the description being made and in order to aid a better understanding of the characteristics of the invention, the present description is accompanied by a set of drawings forming an integral part of the description where, for purposes of illustration and in a non-limiting sense the following is shown:

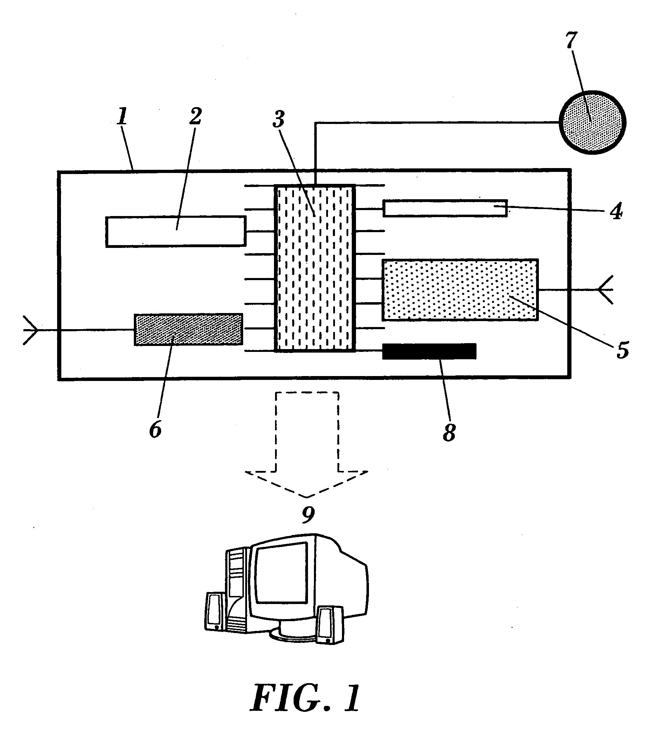

Figure 1 shows a schematic representation of the main components of the system of the invention.

### PREFERRED EMBODIMENT OF THE INVENTION

The system of the invention, as shown in figure 1, is based on the user of the following units:
- a portable and wireless device (1) placed on the body of a person which incorporates the following devices:
   - a biaxial accelerometer (2) that continuously generates two signals along the X and Y axis with levels proportional to the acceleration recorded along those axis; naturally, a triaxial accelerometer can also be used for this function;
   - a microprocessor (3) that includes a fall detection algorithm, the functioning of which is also the object of the invention and will be described further below, as well as an algorithm for analysing the activity of the person;
   - a memory (4) for storing predetermined data;
   - a mobile GSM/GPRS/UMTS communications module that can send SMS messages to a control station or to other receiver units, or any wireless telephony communication module;
   - a GPS type location and global positioning system (6) that determines the position of the person;
   - a button (7) for activating and cancelling the alarm;
   - a battery (8) for supplying power to the device.
   - a remote control system (9) incorporating a receiver that can receive the alarm message sent by the portable device via mobile communication such as GSM/GPRS/UMTS.

The detection device is based on the use of a biaxial accelerometer manufactured using MEMS technology that is composed of two very small silicon plates opposite each other, with the property that one plate is fixed and the other is mounted on a spring, thereby defining a capacitor between the two plates.

When an acceleration occurs the plate mounted on the spring moves toward or away from the fixed plate, changing the capacity of the capacitor proportionally to the movement of the plate and therefore proportionally to the acceleration.

This accelerometer has a small size and weight and a small manufacturing cost.

The method used to detect falls proposed by the invention is based on monitoring the person's activity continuously, for which the acceleration signal is obtained along the X (vertical) and Y (horizontal) axis for the previous 2 seconds, opening a time window that is used as a reference for possible instantaneous events that may occur. If a capture frequency of 500 Hz is used, 1000 individual acceleration values along the X and Y axis can be obtained.

With the values obtained, the microprocessor (3) calculates an average value of the acceleration in this period and uses it to establish maximum limits, differential limits and resting limits used for comparison to the instantaneous acceleration limits, simultaneously performing the two comparisons known as the direct and differential detections that are described further below.

The maximum limits are set at ± 3.5g, with respect to the average acceleration calculated for the Y axis, while the differential limits for the X and Y axis are set at ± 1.6g with respect to the average acceleration calculated for the X axis and the Y axis. The resting limits are set at -0.32g and -1.92g for the X axis, and -0.5g and -1.5g for the Y axis, with respect to the average acceleration calculated for the X axis and the Y axis. These limits are positive and negative as the acceleration can be in a positive or negative sense, depending on the direction of motion. Thus, if a person falls in a similar manner to the front or back, or to the left or right, the same pattern will be detected in the variation of acceleration but in an opposite sense, one positive and the other negative.

The absolute detection is made by comparing the instantaneous acceleration values along the Y axis with the maximum limits, also along the Y axis, so that if the instantaneous acceleration exceeds one of these limits it is determined that a fall has in fact occurred.

Differential detection is made by comparing the instantaneous acceleration values along the X and Y axis so that, if the instantaneous acceleration exceeds one of these limits without reaching the maximum limits in the Y axis, it is determined that a fall has possibly occurred.

In this case, to confirm that the fall has occurred it is checked that the person is lying on the ground and not moving, which would indicate that a fall has in fact taken place. To do so, the instantaneous acceleration values are compared with the resting limits along the X and Y axis, set at -0.32g and -1.92g for the X axis and -0.5g and -1.5g for the Y axis, with respect to the average acceleration calculated for the X axis and the Y axis.

All the limits, both maximum, differential and resting have a tolerance of ±0.5g depending on the sensitivity of the accelerometer.

These absolute and differential limits stored in the memory (4) and the fall detection algorithm programmed in the microprocessor (3) are used to detect falls according to the following stages:
a) the system compares the instantaneous acceleration values along the Y axis to the maximum limits previously determined for the Y axis, with respect to the average calculated for the previous two seconds.
b) If the value of the instantaneous acceleration along the Y axis exceeds the maximum limits, the system determines that a fall has occurred.

Simultaneously, the microprocessor (3) performs a differential analysis of the signal according to the following algorithm:
c) The system compares the instantaneous acceleration values along the X and Y axis to the differential values established previously for the X and Y axis with respect to the average calculated in the previous two seconds.
d) If the value of the instantaneous acceleration along the Y axis exceeds the differential limits but does not reach the maximum limits, the system determines that a possible fall has occurred and opens a new time window for confirming the fall, in which the following operations are performed:
e) For a maximum time of 3 seconds, the instantaneous values along the Y axis acquired by the accelerometer (2) are discarded.
f) After this maximum time of 3 seconds the instantaneous acceleration signals along the X and Y axis are compared to the resting limits for each axis, relative to the last average value calculated.
g) If in the next second none of the acceleration values along the X and Y axis exceed the resting values, the system determines that a fall has occurred.

The alarm button (7) provided in the portable device (1) allows sending an alarm by pressing a button, even when a fall has not occurred or in case of a system failure. In addition, the user can cancel an automatic alarm if the system sends a false alarm or even if a fall has occurred but the user is not harmed.

In short, the method of the invention with the aforementioned equipment has the following functionalities:
- automatic fall detection with a 95% reliability based on using accelerometers with MEMS technology and the detection algorithm of the invention.
- Automatic alarm generation in case of fall.
- Sending alerts by GSM or other type mobile telephony.
- Locating the user by GPS positioning systems.
- Monitoring the person's activity, distinguishing between dynamic activities (such as walking) and passive ones (such as sitting/lying down).
- Generating periodic activity reports.

## Claims

1. A method of monitoring a person's activity and automatic detection of a fall by using a portable and wireless device (1) placed on the person's body, said device (1) comprising an accelerometer (2) for detecting acceleration values along a vertical axis X and a horizontal axis Y, a microprocessor (3) for determining when a fall has occurred, a memory (4) for storing temporary data, a wireless telephony communications module (5) for sending an alarm message to an external receiver of a remote control system (9), a global positioning and location system (6) for indicating the position of the person, an alarm activation and cancellation button (7) and a battery (8), the method comprising the steps of:
- continuously obtaining instantaneous acceleration values along a vertical axis X and a horizontal axis Y for a certain period of time;
- from said instantaneous acceleration values, calculating an average value of acceleration within said period of time;
- based on said calculated average values of acceleration, establishing a first, maximum limit for said horizontal axis Y;
- establishing a second limit for said horizontal axis Y and a third limit for said vertical axis X with respect to said average values of acceleration, said second and third limits being lower than said first, maximum limit;
- comparing the instantaneous acceleration values along said horizontal axis Y with said established first, maximum limit for said horizontal axis Y and, if an instantaneous acceleration value along said horizontal axis Y exceeds said established first, maximum limit for said axis Y, it is unequivocally determined that a fall has occurred; and
- simultaneously comparing the instantaneous acceleration values along said horizontal axis Y and said vertical axis X with said respective established second and third limits for said horizontal axis Y and said vertical axis X and, if an instantaneous acceleration value of one of said two axis exceeds said respective established second and third limits for said axis while said instantaneous acceleration value along said horizontal axis Y is lower than said established first, maximum limit for said horizontal axis Y, it is established that a fall may have occurred.

2. The method according to claim 1, wherein the average value of the acceleration is calculated based on the instantaneous acceleration values obtained in a period of two seconds prior to the determined fall.

3. The method according to claim 2, wherein the first, maximum limit with respect to the average acceleration along the Y axis is set at ±3.5g, while the second limit for the X axis and the third limit for the Y axis are set at ±1.6g, these limits being determined with a variability of ±0.5g depending on the sensitivity of the accelerometer.

4. The method according to any preceding claim, wherein after a fall is detected, an alarm signal is triggered that can be cancelled by auser during a certain time by pressing the button (7), so that after apredetermined time has elapsed without the alarm being cancelled by said user, an alarm message is sent; said user being also capable of triggering said alarm by pressing said button (7) even when a fall has not occurred, or in the case of system failure.

5. The method according to any preceding claim, wherein the acceleration data obtained are processed by the microprocessor (3) using an algorithm that allows identifying the type of activity of a person among a number of preestablished activities stored in the memory (4), obtaining a series of data that are stored in the memory (4) and periodically sent in summarised form to the remote control system (9) having a communications module (5), so that the control system (9) may evaluate the mobility of the person and monitor the person's physical evolution.

6. A portable, wireless device (1) for analysing a person's activity and for automatic fall detection when it is placed on the person's body, which comprises an accelerometer (2) able to detect acceleration values along the X and Y axis, a microprocessor (3) that determines when a fall has occurred, a memory (4) for temporary data storage, a wireless telephony communications module (5) for sending an alarm message to an external receiver of a remote control system (9), a global positioning and location system (6) for indicating the position of the person, an alarm activation and cancellation button (7) and a battery (8), said accelerometer (2) being configured for continuously obtaining instantaneous acceleration values along a vertical axis X and a horizontal axis Y for a certain period of time, said microprocessor (3) being configured for calculating an average value of acceleration within said period of time from said instantaneous acceleration values;
- said microprocessor (3) being configured for, based on said calculated average values of acceleration, establishing a first, maximum limit for said horizontal axis Y;
- said microprocessor (3) being configured for establishing a second limit for said horizontal axis Y and a third limit for saif vertical axis X with respect to said average values of acceleration, said second a third limits being lower than said first, maximum limit;
- said microprocessor (3) being configured for comparing the instantaneous acceleration values along said horizontal axis Y with said established first, maximum limit for said horizontal axis Y and, If an instantaneous acceleration value along said horizontal axis Y exceeds said established first, maximum limit for said axis Y, it is unequivocally determined that a fall has occurred; and
- said microprocessor (3) being configured for simultaneously comparing the instantaneous acceleration values along said horizontal axis Y and said vertical axis X with said respective established second and third limits for said horizontal axis Y and said vertical axis X and, if an instantaneous acceleration value of one of said two axis exceeds said respective established second and third limits for said axis while said instantaneous acceleration value along said horizontal axis Y is lower than said established first, maximum limit for said horizontal axis Y, it is established that a fall may have occurred.

7. The device of either claim 6, wherein said accelerometer (2) is a biaxial accelerometer composed of two silicon plates placed opposite one another, one of them fixed and the other mounted on a spring, so that a capacitor is defined between the two plates.

## Patentansprüche

1. Verfahren zum Überwachen einer Personenaktivität und automatischer Detektion eines Sturzes, unter Verwendung einer tragbaren und drahtlosen Vorrichtung (1), die an dem Körper der Person platziert ist, wobei die Vorrichtung (1) einen Beschleunigungsmesser (2) zum Detektieren von Beschleunigungswerten längs einer Vertikalachse X und einer Horizontalachse Y, einen Mikroprozessor (3) zum Bestimmen, dass ein Sturz eingetreten ist, einen Speicher (4) zum Speichern temporärer Daten, ein Funktelefonkommunikationsmodul (5) zum Aussenden einer Alarmnachricht an einen externen Empfänger eines entfernten Steuersystems (9), ein globales Positionierungs- und Lokalisierungssystem (6) zum Anzeigen der Position der Person, eine Alarmaktivierungs- und Abbruchstaste (7), und eine Batterie (8) umfasst, wobei das Verfahren die Schritte umfasst:
- kontinuierlich Erhalten momentaner Beschleunigungswerte längs einer Vertikalachse X und einer Horizontalachse Y über einen gewissen Zeitraum;
- Berechnen, aus besagten momentanen Beschleunigungswerten, eines Durchschnittswertes von Beschleunigung innerhalb des Zeitraums;
- basierend auf den berechneten Durchschnittswerten von Beschleunigung, Etablieren eines ersten Maximalgrenzwerts für die Horizontalachse Y;
- Etablieren eines zweiten Grenzwerts für die Horizontalachse Y und eines dritten Grenzwerts für die Vertikalachse X in Bezug auf die Durchschnittswerte von Beschleunigung, wobei die zweiten und dritten Grenzwerte niedriger als der erste Maximalgrenzwert sind;
- Vergleichen der momentanen Beschleunigungswerte längs der Horizontalachse Y mit dem ersten etablieren Maximalgrenzwert für die Horizontalachse Y und, falls ein aktueller Beschleunigungswert längs der Horizontalachse Y den etablierten ersten Maximalgrenzwert für die Y-Achse übersteigt, unwiderruflich Feststellen, dass ein Sturz aufgetreten ist; und
- simultan Vergleichen der momentanen Beschleunigungswerte längs der Horizontalachse Y und Vertikalachse X mit den entsprechenden etablierten zweiten und dritten Grenzwerten für die Horizontalachse Y und die Vertikalachse X und, falls ein momentaner Beschleunigungswert einer der zwei Achsen die jeweiligen etablierten zweiten und dritten Grenzwerte für die Achsen übersteigt, während der aktuelle Beschleunigungswert längs der horizontalen Achse Y niedriger als der etablierte erste Maximalgrenzwert für die Horizontalachse Y ist, wird festgestellt, dass ein Sturz aufgetreten sein könnte.

2. Verfahren gemäß Anspruch 1, wobei der Durchschnittswert der Beschleunigung basierend auf den momentanen Beschleunigungswerten berechnet wird, die in einem Zeitraum von zwei Sekunden vor dem festgestellten Sturz erhalten worden sind.

3. Verfahren gemäß Anspruch 2, wobei der erste Maximalgrenzwert in Bezug auf die Durchschnittsbeschleunigung längs der Y-Achse auf ±3,5 g eingestellt wird, während der zweite Grenzwert für die X-Achse und der dritte Grenzwert für die Y-Achse auf ±1,6 g eingestellt sind, wobei diese Grenzwerte mit einer Variabilität von ±0,5 g festgestellt werden, abhängig von der Sensitivität des Beschleunigungsmessers.

4. Verfahren gemäß einem der vorstehenden Ansprüche, wobei, nachdem ein Sturz detektiert worden ist, ein Alarmsignal ausgelöst wird, das von einem Anwender während einer gewissen Zeit durch Drücken der Taste (7) abgebrochen werden kann, so dass nach Verstreichen einer vorgegebenen Zeit ohne Abbrechen des Alarms durch den Anwender eine Alarmnachricht gesendet wird; wobei der Anwender ebenfalls zum Auslösen des Alarmes durch Drücken der Taste (7) in der Lage ist, selbst wenn kein Sturz aufgetreten ist, oder im Fall eines Systemversagens.

5. Verfahren gemäß einem der vorstehenden Ansprüche, wobei die erhaltenen Beschleunigungsdaten vom Mikroprozessor (3) unter Verwendung eines Algorithmus prozessiert werden, der die Identifikation der Art von Aktivität einer Person aus einer Anzahl von vorab etablierten Aktivitäten, die im Speicher (4) gespeichert sind, gestattet, Erhalten einer Reihe von Daten, die im Speicher (4) gespeichert werden, periodisches Senden in summarischer Form an das entfernte Steuersystem (9) mit einem Kommunikationsmodul (5), so dass das Steuersystem (9) die Mobilität der Person evaluieren und die physische Entwicklung der Person überwachen kann.

6. Tragbare Funkvorrichtung (1) zum Analysieren einer Personenaktivität und für automatische Sturz-Detektion, wenn sie an den Körper der Person platziert wird, die umfasst einen Beschleunigungsmesser (2), der zum Detektieren von Beschleunigungswerten längs der X- und Y-Achsen in der Lage ist, einen Mikroprozessor (3), der feststellt, wenn ein Sturz aufgetreten ist, einen Speicher (4) zur vorübergehenden Datenspeicherung, ein Funktelefonkommunikationsmodul (5) zum Aussenden einer Alarmnachricht an einen externen Empfänger eines entfernten Steuersystems (9), ein globales Positionierungs- und Lokalisierungssystem (6) zum Anzeigen der Position der Person, eine Alarmaktivierungs- und Abbruchstaste (7) und eine Batterie (8), wobei der Beschleunigungsmesser (2) dafür konfiguriert ist, kontinuierlich momentane Beschleunigungswerte längs einer Vertikalachse X und einer Horizontalachse Y über einen gewissen Zeitraum zu erhalten, wobei der Mikroprozessor (3) dafür konfiguriert ist, einen Durchschnittswert an Beschleunigung innerhalb des Zeitraums aus den momentanen Beschleunigungswerten zu berechnen;
- wobei der Mikroprozessor (3) dafür konfiguriert ist, basierend auf den berechneten Durchschnittswerten der Beschleunigung einen ersten Maximalgrenzwert für die horizontale Y-Achse zu etablieren;
- der Mikroprozessor (3) dafür konfiguriert ist, einen zweiten Grenzwert für die Horizontalachse Y und einen dritten Grenzwert für die Vertikalachse X in Bezug auf die Durchschnittswerte an Beschleunigung zu etablieren, wobei die zweiten und dritten Grenzwerte niedriger als der erste Maximalgrenzwert sind;
- der Mikroprozessor (3) zum Vergleichen der momentanen Beschleunigungswerte längs der Horizontalachse Y mit dem ersten etablierten Maximalgrenzwert für die Horizontalachse Y konfiguriert ist, und, falls ein momentaner Beschleunigungswert längs der Horizontalachse Y den etablierten ersten Maximalgrenzwert für die Achse Y übersteigt, unwiderruflich festgestellt wird, dass ein Sturz aufgetreten ist; und
- der Mikroprozessor (3) für simultanes Vergleichen der momentanen Beschleunigungswerte längs der Horizontalachse Y und der Vertikalachse X in Bezug auf entsprechende etablierte zweite und dritte Grenzwerte für die Horizontalachse Y und die Vertikalachse X konfiguriert ist, und, falls ein momentaner Beschleunigungswert einer der zwei Achsen die entsprechenden etablierten zweiten und dritten Grenzwerte für die Achsen übersteigt, während der aktuelle Beschleunigungswert längs der Horizontalachse Y niedriger als der etablierte erste Maximalgrenzwert für die Horizontalachse Y ist, festgestellt wird, dass ein Sturz aufgetreten sein könnte.

7. Vorrichtung nach Anspruch 6, wobei der Beschleunigungsmesser (2) ein biaxialer Beschleunigungsmesser ist, der aus zwei Siliziumplatten besteht, die einander gegenüberliegend platziert sind, von denen eine fixiert und die andere auf einer Feder montiert ist, so dass ein Kondensator zwischen den zwei Platten definiert ist.

## Revendications

1. Procédé de surveillance de l'activité d'une personne et de détection automatique d'une chute par l'utilisation d'un dispositif portable et sans fil (1) placé sur le corps de la personne, ledit dispositif (1) comprenant un accéléromètre (2) pour détecter des valeurs d'accélération suivant un axe vertical X et un axe horizontal Y, un microprocesseur (3) pour déterminer quand une chute a lieu, une mémoire (4) pour stocker des données temporaires, un module de communication téléphonique sans fil (5) pour envoyer un message d'alarme à un récepteur externe d'un système de commande à distance (9), un système global de positionnement et de localisation GPS (6) pour indiquer la position de la personne, un bouton d'activation et d'annulation d'alarme (7) et une batterie (8), le procédé comprenant les étapes consistant à :
obtenir de manière continue des valeurs d'accélération instantanée suivant un axe vertical X et un axe horizontal Y pour une certaine période de temps ;
à partir desdites valeurs d'accélération instantanée, calculer une valeur d'accélération moyenne pendant ladite période de temps ;
sur la base desdites valeurs d'accélération moyenne calculées, établir une première limite maximale pour ledit axe horizontal Y ;
établir une deuxième limite pour ledit axe horizontal Y et une troisième limite pour ledit axe vertical X par rapport auxdites valeurs d'accélération moyenne, lesdites deuxième et troisième limites étant inférieures à ladite première limite maximale ;
comparer les valeurs d'accélération instantanée suivant ledit axe horizontal Y avec ladite première limite maximale établie pour ledit axe horizontal Y et, si une valeur d'accélération instantanée suivant ledit axe horizontal Y dépasse ladite première limite maximale établie pour ledit axe Y, il est déterminé de manière univoque qu'une chute a eu lieu ; et
comparer simultanément les valeurs d'accélération instantanée suivant ledit axe horizontal Y et ledit axe vertical X avec lesdites deuxième et troisième limites respectives établies pour ledit axe horizontal Y et ledit axe vertical X et, si une valeur d'accélération instantanée de l'un des deux dits axes dépasse lesdites deuxième et troisième limites respectives établies pour lesdits axes alors que ladite valeur d'accélération instantanée suivant ledit axe horizontal Y est inférieure à ladite première limite maximale établie pour ledit axe horizontal Y, il est établi qu'une chute peut avoir eu lieu.

2. Procédé selon la revendication 1, dans lequel la valeur moyenne de l'accélération est calculée sur la base des valeurs d'accélération instantanée obtenues dans une période de deux secondes avant la chute déterminée.

3. Procédé selon la revendication 2, dans lequel la première limite maximale par rapport à l'accélération moyenne suivant l'axe Y est fixée à ± 3,5 g, alors que la deuxième limite pour l'axe X et la troisième limite pour l'axe Y sont fixées à ± 1,6 g, ces limites étant déterminées avec une variabilité de ± 0,5 g selon la sensibilité de l'accéléromètre.

4. Procédé selon l'une quelconque des revendications précédentes, dans lequel une fois qu'une chute a été détectée, un signal d'alarme est déclenché qui peut être annulé par un utilisateur durant un certain temps en pressant le bouton (7), de sorte qu'après qu'un temps prédéterminé se soit écoulé sans que l'alarme ait été annulée par ledit utilisateur, un message d'alarme est envoyé ; ledit utilisateur étant également capable de déclencher ladite alarme en pressant ledit bouton (7) même quand une chute n'a pas eu lieu, ou dans le cas d'un défaut du système.

5. Procédé selon l'une quelconque des revendications précédentes, dans lequel les données d'accélération obtenues sont traitées par le microprocesseur (3) en utilisant un algorithme qui permet d'identifier le type d'activité d'une personne parmi un nombre d'activités préétablies stockées dans la mémoire (4), d'obtenir une série de données qui sont stockées dans la mémoire (4) et envoyées périodiquement sous forme résumée au système de commande à distance (9) ayant un module de communication (5), de sorte que le système de commande (9) puisse évaluer la mobilité de la personne et surveiller l'évolution physique de la personne.

6. Dispositif portable sans fil (1) pour analyser l'activité d'une personne et pour la détection automatique d'une chute quand il est placé sur le corps de la personne, qui comprend un accéléromètre (2) capable de détecter des valeurs d'accélération suivant les axes X et Y, un microprocesseur (3) qui détermine quand une chute a eu lieu, une mémoire (4) pour le stockage temporaire de données, un module de communication téléphonique sans fil (5) pour envoyer un message d'alarme à un récepteur externe d'un système de commande à distance (9), un système global de positionnement et de localisation GPS (6) pour indiquer la position de la personne, un bouton d'activation et d'annulation d'alarme (7) et une batterie (8), ledit accéléromètre (2) étant configuré pour obtenir de manière continue des valeurs d'accélération instantanée suivant un axe vertical X et un axe horizontal Y pendant une certaine période de temps, ledit microprocesseur (3) étant configuré pour calculer une valeur d'accélération moyenne pendant ladite période de temps à partir desdites valeurs d'accélération instantanée ;
ledit microprocesseur (3) étant configuré pour, sur la bases desdites valeurs d'accélération moyenne calculées, établir une première limite maximale pour ledit axe horizontal Y ;
ledit microprocesseur (3) étant configuré pour établir une deuxième limite pour ledit axe horizontal Y et une troisième limite pour ledit axe vertical X par rapport auxdites valeurs d'accélération moyenne, lesdites deuxième et troisième limites étant inférieures à ladite première limite maximale ;
ledit microprocesseur (3) étant configuré pour comparer les valeurs d'accélération instantanée suivant ledit axe horizontal Y avec ladite première limite maximale établie pour ledit axe horizontal Y et, si une valeur d'accélération instantanée suivant ledit axe horizontal Y dépasse ladite première limite maximale établie pour ledit axe Y, il est déterminé de manière univoque qu'une chute a eu lieu ; et
ledit microprocesseur (3) étant configuré pour comparer simultanément les valeurs d'accélération instantanée suivant ledit axe horizontal Y et ledit axe vertical X avec lesdites deuxième et troisième limites respectives établies pour ledit axe horizontal Y et ledit axe vertical X et, si une valeur d'accélération instantanée de l'un des deux dits axes dépasse lesdites deuxième et troisième limites respectives établies pour lesdits axes alors que ladite valeur d'accélération instantanée suivant ledit axe horizontal Y est inférieure à ladite première limite maximale établie pour ledit axe horizontal Y, il est établi qu'une chute peut avoir eu lieu.

7. Dispositif selon la revendication 6, dans lequel ledit accéléromètre (2) est un accéléromètre biaxial composé de deux plaques de silicium placées de manière opposée l'une à l'autre, l'une d'elle étant fixée et l'autre étant montée sur un ressort, de sorte qu'un condensateur est défini entre les deux plaques.
